# FASCICULE DE BREVET EUROPEEN

(11) **EP 1 857 133 B1**
(45) Date de publication et mention de la délivrance du brevet: **06.03.2013**
(21) Numéro de dépôt: 07301045.6
(22) Date de dépôt: 16.05.2007
(51) Int. Cl.: A61M 15/00

(54) **Appareils générateur d'aérosols médicaux incluant un dispositif d'amélioration de l'efficacité de dépôt d'aérosol**
Geräte zur Erzeugung medizinischer Aerosole, die eine Vorrichtung zur Verbesserung der Effizienz der Aerosolablagerung enthalten
Appliances for generating medical aerosols including a device for improving aerosol deposit efficiency

(30) Priorité: 16.05.2006 FR 0651761
(43) Date de publication de la demande: 21.11.2007
(73) Titulaire: LA DIFFUSION TECHNIQUE FRANCAISE, 42000 Saint Etienne (FR)
(72) Inventeur: Vecellio-None, Laurent, 31170, Chambray les Tours (FR); Massardier, Michel, 42000, Saint Etienne (FR); Chantrel, Gilles, 42100, Saint-Etienne (FR)
(74) Mandataire: Dupuis, François

(56) Documents cités:
- EP-A- 1 674 122
- WO-A-02/13896
- FR-A1- 2 783 431
- US-A- 4 819 629
- US-A- 6 138 668

## Description

L'invention se rattache au secteur technique des systèmes de génération d'aérosols médicaux.

Les appareils de génération d'aérosols médicaux ont pour fonction de transformer un liquide médicamenteux sous forme d'aérosol pour être administré dans les voies aériennes respiratoires.

Divers appareils générateurs d'aérosols médicaux existent sur le marché. Ce sont des générateurs pneumatiques, ultra soniques, à membrane, notamment, ainsi que des flacons pressurisés avec valve doseuse.

Le Déposant est l'un des leaders dans la fabrication et la commercialisation de ce type d'appareils. Malgré tous les efforts de recherche en rapport avec ces appareils, générateurs, nébuliseurs, flacons pressurisés, les différentes études publiées en rapport sont toutes d'accord pour constater qu'une large part de l'aérosol produit est perdue, gaspillée et ne profite pas au traitement thérapeutique souhaité. La part d'utilisation de l'aérosol dans sa fonction est ainsi estimée par les auteurs et fabricants à 25 % environ, ce qui est couramment explicité par la fraction inhalable ou disponible. La déperdition est due à plusieurs causes dont la perte de médicament dans l'atmosphère durant la phase expiratoire du patient. L'explication qui suit est la conséquence directe d'un problème issu des résultats d'une étude in vivo concernant la demande de brevet FR 04 13784 appartenant au présent Demandeur.

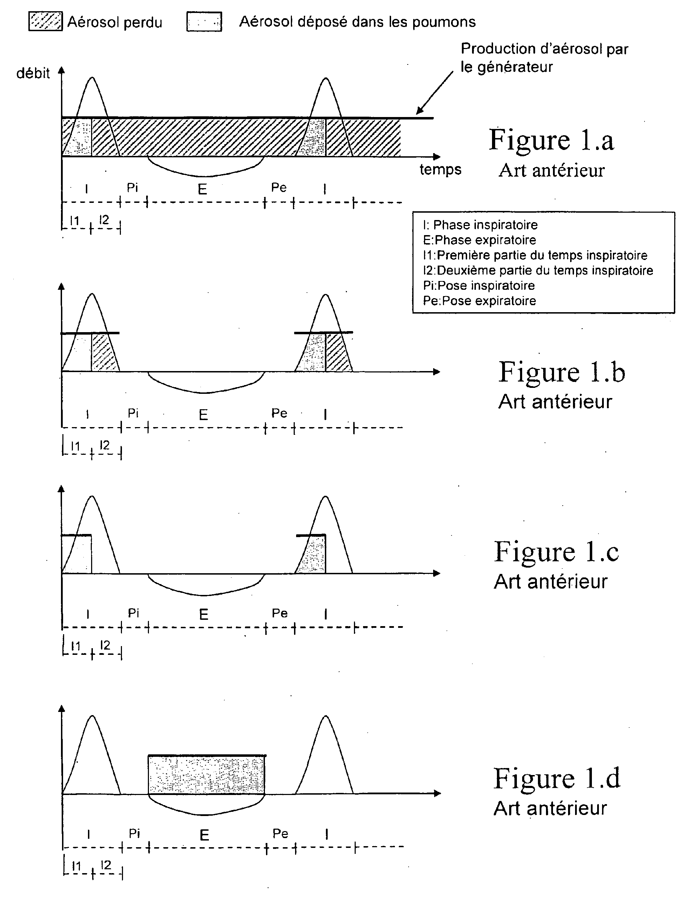

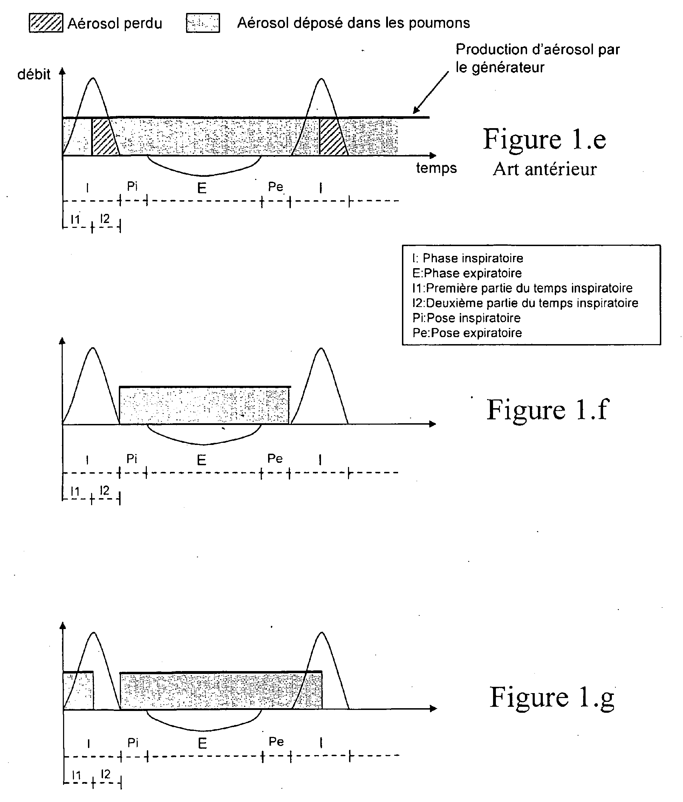

Certains appareils générateurs d'aérosol médicamenteux qui produisent un aérosol en continu peuvent générer une perte de médicament dans l'atmosphère durant la phase expiratoire (E) du patient. En effet, lorsque le patient expire dans l'appareil générateur, ou n'inspire pas dans l'appareil générateur, l'aérosol peut s'échapper librement de l'appareil générateur. C'est le cas par exemple des nébuliseurs pneumatiques fonctionnant en continu, de certains nébuliseurs ultrasoniques et de certains nébuliseurs à membrane (Figure 1.a)

Pour palier au problème de perte d'aérosol durant la phase expiratoire, différents appareils proposent de ne produire l'aérosol que durant la phase inspiratoire (I) du patient (figure 1b). Ces appareils peuvent faire appel à des commandes électroniques basées sur des mesures en temps réel de pression ou de débit d'air induits par le patient. D'autres appareils utilisent des méthodes mécaniques directement liées à la source de génération de l'aérosol. Par exemple, avec l'utilisation de ressort se rétractant lors de la dépression induite par l'inspiration (I) du patient ayant pour effet l'abaissement d'un gicleur de liquide permettant la génération de l'aérosol. Tous ces appareils produisent l'aérosol durant la totalité de l'inspiration du patient. Ils améliorent le rendement des appareils et peuvent être destinés à contrôler la dose de médicament produite sous forme aérosol, comme par exemple dans le cas des tests de provocation bronchique.

L'inconvénient que pose ces appareils est que même en limitant la production de l'aérosol durant l'inspiration (I), une partie de l'aérosol est perdue dans l'atmosphère. Cette perte n'est plus la conséquence des fuites dues à l'appareil, elle est uniquement due aux propriétés anatomiques des voies respiratoires de l'homme.

Les voies respiratoires humaines sont classiquement décomposées en deux grandes régions : la région de conduction de l'air et la région respiratoire. La région de conduction s'étend de la bouche jusqu'aux bronches et elle a pour rôle comme son nom l'indique de conduire l'air. Cette région est composée de conduits de large section limitant le dépôt de l'aérosol. La région respiratoire s'étend des bronches jusqu'aux alvéoles pulmonaires et elle a pour rôle comme son nom l'indique d'effectuer les échanges gazeux. Cette région est composée de petits conduits débouchant sur des sacs alvéolaires. Cette région respiratoire favorise le dépôt de l'aérosol inhalé. Lorsque le patient inhale un certain volume d'air (Vt), celui-ci peut être décomposé en deux parties. Le volume alvéolaire (V1) pénétrant dans la région respiratoire (RR) et le volume mort (V2) pénétrant dans la région de conduction (RC). La première partie de l'air inhalé est le volume alvéolaire (V1), la deuxième partie de l'air inhalé est le volume mort (V2). Par exemple, si le patient inhale 500ml d'air et qu'il possède un volume mort anatomique de 200ml (V2), les 300 premiers ml d'air inhalé (V1) traverseront la région de conduction (RC) puis iront dans la région respiratoire (RR). Les derniers 200ml inhalés (V2) s'arrêteront dans la région de conduction (RC). Il en est de même avec les aérosols inhalés. Lorsque le patient inhalera 500ml d'un volume d'air rempli d'un aérosol homogène, les premiers 300ml d'aérosols (V1) iront dans la région respiratoire (RR) et auront une forte efficacité de dépôt. Les derniers 200 ml d'aérosols (V2) pénétreront dans la région de conduction (RC) et auront une faible efficacité de dépôt. L'aérosol non déposé dans les voies respiratoires sera exhalé par le patient. Pour limiter cette perte d'aérosol exhalé, l'art antérieur propose que la première partie du volume d'air inhalé (V1) soit chargée d'aérosol et que la deuxième partie du volume d'air inhalé (V2) soit dépourvue d'aérosol. Cette idée actuellement exploitée par différents appareils est mise en oeuvre par différentes solutions techniques.

La première solution technique consiste à produire l'aérosol uniquement durant la première partie du temps inspiratoire (I1) du patient (Figure 1c). Ainsi, lorsque le patient inhale un volume Vt, la première partie du volume inhalé est chargée d'aérosol et la deuxième partie du volume inhalé est dépourvue d'aérosol. Ce système sophistiqué est basé sur l'enregistrement de plusieurs cycles respiratoires et prédit la durée optimale de la génération de l'aérosol pour l'inspiration suivante du patient. Les durées de génération d'aérosol excessivement courtes durant une partie de la phase inspiratoire ont pour conséquence des durées de traitements longues (plus de 30 min pour 1ml de médicament). Ceci est dû en grande partie au fait que le temps expiratoire est toujours plus long que le temps inspiratoire (en général un facteur 2).

La deuxième solution technique consiste à produire l'aérosol uniquement durant la phase expiratoire (E) du patient (US4819629) (Figure 1d). Dans cette configuration, le système de génération d'aérosol est composé d'un circuit inspiratoire distinct du circuit expiratoire. Le générateur d'aérosol est connecté sur le circuit inspiratoire et est asservi par l'intermédiaire d'un capteur de débit. Dans ces conditions, lors de l'expiration du patient, l'aérosol est produit et stocké dans le circuit inspiratoire. Lors de la phase inspiratoire, le générateur d'aérosol ne produit plus d'aérosol et l'aérosol stocké dans le circuit inspiratoire est inhalé par le patient. Ainsi, lorsque le patient inhale un volume Vt plus important que le volume de stockage de l'aérosol, la première partie du volume inhalé est chargée d'aérosol et la deuxième partie du volume inhalé est dépourvue d'aérosol. Ce système possède l'avantage d'une durée de traitement plus courte que le système synchronisé à l'inspiration mais son fonctionnement est imparfait pour la nébulisation chez le patient. Par exemple, lorsque l'inhalation a lieu à l'aide d'un embout buccal, le patient à la possibilité d'expirer par le nez. Dans ces conditions, l'air expiré par le patient n'est pas détecté par le capteur de débit et l'aérosol n'est pas produit.

En conclusion, les appareils de génération d'aérosol produisant l'aérosol uniquement durant la totalité de la phase inspiratoire (I) du patient limitent les pertes sous forme de fuite d'aérosol due à l'appareil mais ne limitent pas les pertes d'aérosol par exhalation (aérosol pénétrant et ressortant des voies respiratoires). Les appareils de génération d'aérosol produisant l'aérosol uniquement durant la première partie de la phase inspiratoire (I1) améliorent l'efficacité de dépôt de l'aérosol en limitant les pertes d'aérosol exhalé mais augmentent la durée des séances d'inhalation (débit d'aérosol réduit). Les appareils d'aérosol produisant l'aérosol uniquement durant la phase expiratoire (E) améliorent l'efficacité de dépôt de l'aérosol (moins d'aérosol exhalé) mais ne fonctionnent pas dans toutes les situations.

Une autre solution pour limiter la perte d'aérosol sans augmenter la durée de la séance consiste à adjoindre au générateur une zone de stockage. L'aérosol produit pendant l'expiration est stocké dans la zone de stockage pour être disponible à l'inhalation suivante. Ce principe est appliqué dans la demande de brevet FR2879465 du demandeur. Il présente néanmoins l'inconvénient de ne pas limiter la perte d'aérosol exhalé (Fig. 1^{e}).

La démarche du Demandeur a donc été de reconsidérer le problème de cette perte de médicament exhalé et de rechercher une nouvelle solution technique afin d'améliorer les conditions de traitement thérapeutique des patients en terme de durée de traitement.

Face à cette situation, le Demandeur s'est alors orienté sur une conception différente de ce type d'appareils.

Selon une première caractéristique de l'invention, l'appareil générateur d'aérosol pour liquide à fonctionnement automatique agencé pour améliorer l'efficacité de dépôt d'aérosol, comprenant :
- un générateur d'aérosol (GA) de liquide sous forme de nébuliseur produisant un aérosol de façon continue
- une zone de stockage (ZS) de l'aérosol.
- une zone de transfert (ZT) de l'aérosol vers le patient,
- un dispositif à fonctionnement automatique permettant la génération de l'aérosol et son transfert dans la zone de stockage uniquement durant les phases de pauses inspiratoires, les phases expiratoires et les phases de pauses expiratoires ; et par l'arrêt de la génération de l'aérosol par le générateur d'aérosols dans la zone de stockage durant la totalité de l'inspiration du patient dans le système,
- une zone de stockage de l'aérosol produit contenant au minimum deux ouvertures dont l'une permet au minimum le passage de l'air et l'autre le passage de l'aérosol depuis la zone de stockage vers le patient n'autorisant pas le passage de l'air expiré par le patient à travers la zone de stockage,
- le dispositif étant un capteur placé sur le circuit inspiratoire de l'air induit par le patient et relié au générateur d'aérosol par un boîtier d'alimentation, ledit capteur ayant pour fonction d'identifier les phases d'inspiration pour produire l'aérosol dans la zone de stockage, durant la totalité de la phase de non inspiration du patient dans l'appareil et l'arrêt de la production d'aérosol lors de la totalité de l'inspiration du patient dans l'appareil.

Ces caractéristiques et d'autres encore ressortiront bien de la suite de la description.
- Les figures 1a, 1b, 1c, 1d, 1e, montrent de manière schématique le fonctionnement, selon l'art antérieur, des appareils connus avec les phases d'inspiration (I) et d'expiration (E).
- La figure 1f montre la mise en oeuvre de l'invention avec les phases précitées avec la génération de l'aérosol durant la pause inspiratoire (Pi), la phase expiratoire (E) et la pause expiratoire (Pe) et l'arrêt de la génération de l'aérosol durant la totalité de la phase inspiratoire (I).
- La figure 1g montre une mise en oeuvre non couverte par l'invention avec les phases précitées avec la génération de l'aérosol durant la pause inspiratoire (Pi), la phase expiratoire (E), la pause expiratoire (Pe) et la première partie de la phase inspiratoire (I1) ; et l'arrêt de la génération de 1 'aérosol durant la deuxième partie de la phase inspiratoire (12).
- La figure 2 illustre le principe de base de l'invention en vue de section lors de la non inspiration du patient dans l'appareil
- Les figures 3.1, 3.2, 3.3 illustrent le premier principe de l'invention en vue de section lors de la cinétique inspiratoire du patient, selon les phases t1, t2, t3 de fonctionnement et selon la mise en oeuvre de la figure 1f.
- Les figures 4.1, 4.2, 4,3 illustrent le deuxième principe de l'invention en vue de section lors de la cinétique inspiratoire du patient, selon les phases t1, t2, t3 de fonctionnement, quand l'aérosol est encore produit en début d'inspiration, selon la mise en oeuvre de la figure 1g et augmentant le dépôt d'aérosol dans la zone de respiration RR.
- Les figures 5 et 6 sont des vues de l'appareil incluant le dispositif d'amélioration de l'efficacité de dépôt de l'aérosol et concernent l'arrêt de la production de l'aérosol uniquement lorsque le patient inspire dans le système à valve unique.
- Les figures 7, 8 et 9 sont des vues de l'appareil incluant le dispositif d'amélioration de l'efficacité de dépôt de l'aérosol et concernent l'arrêt de la production de l'aérosol lors de la deuxième partie de la phase inspiratoire du patient dans le système à valve unique.
- Les figures 10 et 11 sont des vues de l'appareil incluant le dispositif d'amélioration de l'efficacité de dépôt de l'aérosol et concernent l'arrêt de la production de l'aérosol lors de la totalité de la phase inspiratoire du patient dans le système à double valve avec le capteur de pression placé sur la zone de transport ZT.
- Les figures 12, 13 et 14 sont des vues non couvertes par l'invention de l'appareil incluant le dispositif d'amélioration de l'efficacité de dépôt de l'aérosol et concernent l'arrêt de la production de l'aérosol lors de la deuxième partie de la phase inspiratoire du patient dans le système à double valve.
- Les figures 15 et 16 sont des vues de l'appareil incluant le dispositif d'amélioration de l'efficacité de dépôt de l'aérosol et concernent l'arrêt de la production de l'aérosol lors de la totalité de la phase inspiratoire du patient dans le système à double valve avec le capteur de pression placé sur la ZS.
- Les figures 17 et 18 sont des vues de l'appareil incluant le dispositif d'amélioration de l'efficacité de dépôt de l'aérosol et concernent l'arrêt de la production de l'aérosol lors de la totalité de la phase inspiratoire du patient dans le système à valve unique avec filtre.
- Les figures 19 et 20 sont des vues de l'appareil incluant le dispositif d'amélioration de l'efficacité de dépôt de l'aérosol, et concernent l'arrêt de la production de l'aérosol, durant la phase inspiratoire, lorsque le patient est ventilé mécaniquement par un respirateur.
- Les figures 21, 22 et 23 sont des vues de l'appareil non couvert par l'invention incluant le dispositif d'amélioration de l'efficacité de dépôt de l'aérosol, et concernent l'arrêt de la production de l'aérosol durant deuxième partie de la phase inspiratoire, lorsque le patient est ventilé mécaniquement par un respirateur

La respiration du patient peut être décomposée en différentes phases : La phase inspiratoire correspondant à la pénétration de l'air extérieur dans les voies respiratoires du patient, la pause inspiratoire correspondant à une pause de la respiration du patient à la fin de son inspiration, la phase expiratoire correspondant à l'évacuation de l'air contenu dans les voies respiratoires vers l'extérieur du patient et la pause expiratoire correspondant à une pause de la respiration du patient à la fin de son expiration (Fig 1 a).

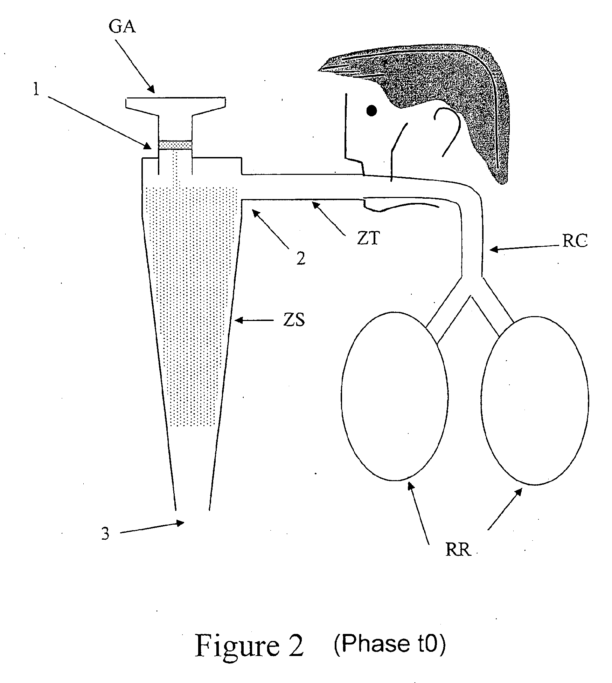

Les appareils de génération d'aérosol médicaux sont composés d'un générateur d'aérosol à proprement dit et d'une interface entre le générateur d'aérosol et le patient. Le générateur d'aérosol (GA) est la source de génération de l'aérosol. L'interface patient - générateur d'aérosol permet le transport de l'aérosol, depuis le générateur vers le patient (exemples : masque, embout buccal, embout nasal, circuit de ventilateur mécanique, sonde d'intubation, sonde trachéale...), cette zone peut être appelée zone de transport (ZT). Selon l'invention, une zone intermédiaire est ajoutée entre le générateur d'aérosol (GA) et la zone de transport (ZT). Cette zone intermédiaire, appelée zone de stockage (ZS) est destinée et a pour fonction de stocker l'aérosol produit. L'invention est basée sur le principe de la production de l'aérosol par le générateur d'aérosol (GA) dans la zone de stockage (ZS) durant les phases de pauses inspiratoires, les phases expiratoires et les phases de pauses expiratoires (Figure 2). L'appareil selon l'invention permettant une amélioration de l'efficacité de dépôt d'aérosol contient une zone de stockage (ZS) et un dispositif permettant le contrôle et la commande de la production de l'aérosol. Ce dispositif peut faire partie intégrante de l'appareil de génération de l'aérosol ou être dissocié.

La zone de stockage (ZS) possède au minimum deux ouvertures (2, 3) et présente une contenance de préférence inférieure à 500ml. La première ouverture (3) permet au minimum le passage de l'air depuis l'extérieur de la zone de stockage (ZS) vers l'intérieur de la zone de stockage (ZS). La deuxième ouverture (2) permet le passage et l'évacuation de l'aérosol depuis l'intérieur de la zone de stockage (ZS) vers l'extérieur de la zone de stockage (ZS). Ces ouvertures peuvent être disposées de façon quelconque sur les différentes faces de la zone de stockage (ZS). Ces ouvertures peuvent être connectées à différents éléments laissant passer l'air ou l'aérosol (valves, zone de transport, filtre,... ). Dans le cas ou le générateur d'aérosol (GA) permet le passage de l'air depuis l'extérieur de la zone de stockage (ZS) vers l'intérieur de la zone de stockage (ZS), il peut être connecté à la première ouverture de la zone de stockage (ZS). Le générateur d'aérosol (GA) peut être également connecté à une troisième ouverture de la zone de stockage (ZS) ou encore être contenu dans la zone de stockage (ZS).

Le dispositif permet la production automatique de l'aérosol par le générateur d'aérosol (GA) dans la zone de stockage (ZS) lors des phases expiratoires (E) et des phases de pauses inspiratoires (Pi) et phases de pauses expiratoires (Pe) du patient dans l'appareil (figure 1f). Le dispositif permet également l'arrêt automatique de la production de l'aérosol par le générateur d'aérosol (GA) dans la zone de stockage (ZS) lors de la totalité des phases inspiratoires (I, figure 1f). Ce dispositif peut être par exemple un capteur de pression relié à un système électrique, lui-même raccordé au générateur d'aérosol (GA). Ce dispositif peut être également un capteur de débit relié à un système électrique raccordé au générateur d'aérosol (GA). Ce dispositif peut être un élément mécanique contenu dans le générateur d'aérosol (GA) ou relié au générateur d'aérosol (GA). Ce dispositif est connecté sur le circuit inspiratoire du système de génération d'aérosol. Le circuit inspiratoire est défini par le volume contenant l'air ou l'aérosol dirigé vers le patient durant la phase inspiratoire. Les générateurs d'aérosol (GA) pouvant être utilisés dans ce dispositif ne concernent que les générateurs d'aérosol (GA) produisant un aérosol de façon continue. On entend par continu, un aérosol ne délivrant pas de bouffées d'aérosols ; ceci par opposition aux aérosols flacons pressurisés avec valve doseuse. La notion de production continue par un générateur d'aérosol (GA) peut être définie comme la production d'un aérosol tout au long d'un cycle respiratoire (inspiration + expiration + pauses respiratoires). Les générateurs d'aérosol (GA) concernés sont par exemple les nébuliseurs pneumatiques, les nébuliseurs ultrasoniques, les nébuliseurs à membrane.

A partir de ce principe, différentes configurations de mise en oeuvre de l'appareil incluant le dispositif de production et de transfert d'aérosols peuvent être envisagées, l'aérosol pouvant être à base de liquide ou de poudre selon les configurations.

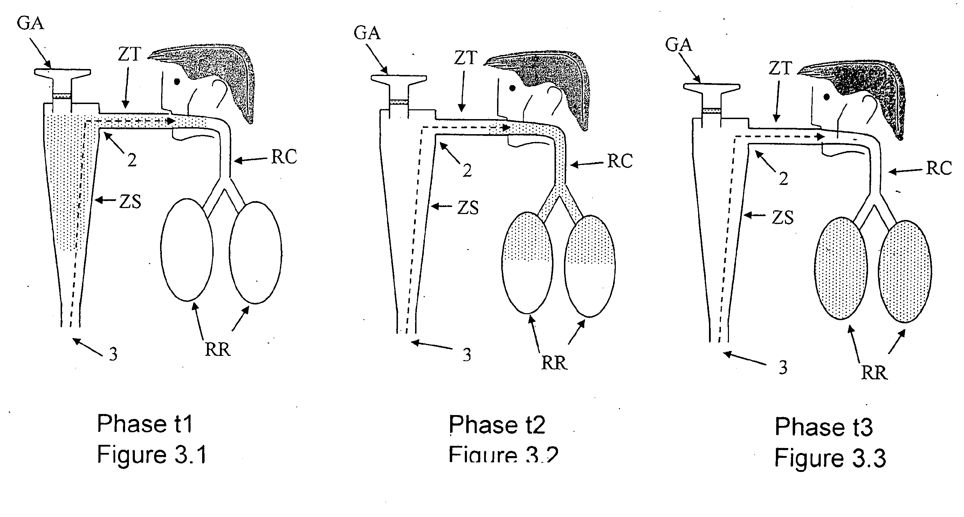

Ainsi selon l'invention et dans son principe de mise en oeuvre, l'appareil est composé d'un générateur d'aérosol produisant, par exemple, l'aérosol durant la non inspiration du patient et d'une zone de stockage (300 ml par exemple) possédant trois ouvertures (figures 2 et 3). La première ouverture (1) est destinée à recevoir le générateur d'aérosol (GA), la deuxième ouverture (2) est destinée à recevoir la zone de transport (ZT) et la troisième ouverture à ne rien recevoir. La zone de transport (ZT) est une pièce cylindrique permettant le transport de l'aérosol depuis la zone de stockage (ZS) vers la bouche du patient.

Dans un premier exemple, durant la phase expiratoire et les phases de pauses respiratoires, l'aérosol est produit par le générateur d'aérosol (GA) dans le volume V1 de la zone de stockage (ZS) (Figure 2).

Durant la phase inspiratoire, c'est-à-dire lorsque le patient inspire un volume d'air (Vt), le générateur d'aérosol (GA) ne produit plus d'aérosol (Figure 3.1). Au début de la phase inspiratoire (Figure 3.1, phase t1) l'air induit par l'inspiration pénètre dans la zone de stockage (ZS) par l'ouverture 3 et déplace le volume Vl d'aérosol à travers l'ouverture (2) vers la bouche du patient par l'intermédiaire de la zone de transport (ZT). A mi-temps de la phase inspiratoire du patient (Figure 3.2, phase t2), la zone de stockage (ZS) a été vidée de l'aérosol, elle ne contient désormais plus que de l'air sans aérosol. Le volume V1 d'aérosol est alors contenu dans la région de conduction (RC) du patient et dans une partie de la région respiratoire (RR) du patient. Durant la fin de l'inspiration (Figure 3.3, phase t3), le reste du volume d'air inspiré par le patient est dépourvu d'aérosol (V2=Vt-V1). Cet air « propre » remplit la région de conduction (RC) du patient et pousse l'aérosol dans la région respiratoire (RR) du patient. L'aérosol est alors uniquement contenu dans la région respiratoire (RR) du patient, région correspondante à l'efficacité de dépôt maximale en comparaison de la région de conduction (RC). La proportion d'aérosol exhalé s'en trouve donc diminuée.

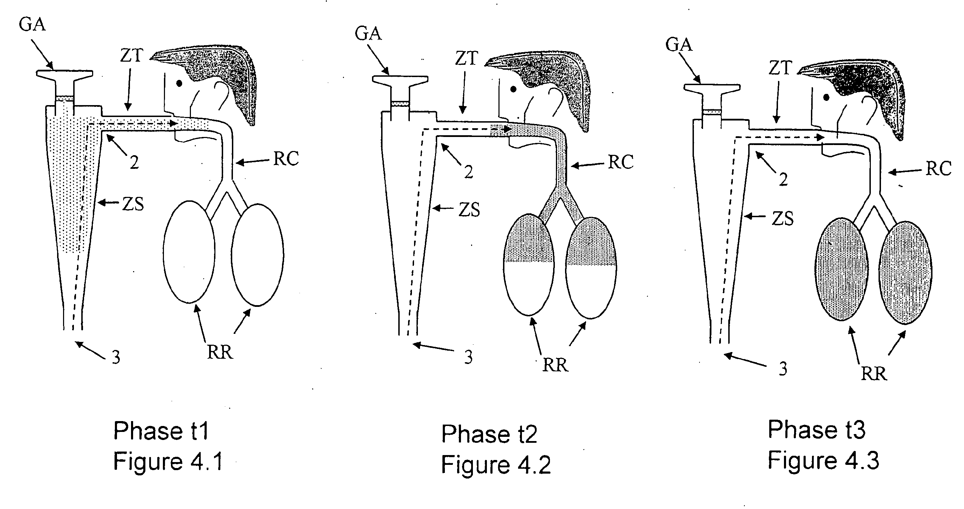

Dans un deuxième exemple, durant la phase expiratoire et durant les phases de pauses respiratoires, l'aérosol est produit par le générateur d'aérosol (GA) dans le volume V1 de la zone de stockage (ZS) (Figure 4.1).

Au début de la phase inspiratoire (Figure 4.1, phase t1), le générateur d'aérosol (GA) produit l'aérosol dans la ZS augmentant ainsi la quantité d'aérosol inhalé et l'air induit par l'inspiration pénètre dans la zone de stockage (ZS) par l'ouverture 3 et déplace le volume V1 d'aérosol à travers l'ouverture (2) vers la bouche du patient par l'intermédiaire de la zone de transport (ZT). A mi-temps de la phase inspiratoire du patient (Figure 4.2, phase t2), le générateur d'aérosol (GA) ne produit plus d'aérosol et la zone de stockage (ZS) a été vidée de l'aérosol, elle ne contient désormais plus que de l'air sans aérosol médical. Le volume V1 d'aérosol est alors contenu dans la région de conduction (RC) du patient et dans une partie de la région respiratoire (RR) du patient. Durant la fin de l'inspiration (Figure 4.3, phase t3), le générateur d'aérosol (GA) ne produit plus d'aérosol et le reste du volume d'air inspiré par le patient est dépourvu d'aérosol (V2=Vt-V1). Cet air « propre » remplit la région de conduction (RC) du patient et pousse l'aérosol dans la région respiratoire (RR) du patient. L'aérosol est alors uniquement contenu dans la région respiratoire (RR) du patient, région correspondante à l'efficacité de dépôt maximale en comparaison de la région de conduction (RC). La proportion d'aérosol exhalé s'en trouve donc diminuée et la quantité d'aérosol inhalé est augmentée. En comparaison du premier exemple, ce deuxième exemple permet d'augmenter pour chaque cycle respiratoire la quantité d'aérosol déposé dans les poumons, ceci est représenté par une matérialisation en gris foncé du dépôt de l'aérosol dans les poumons.

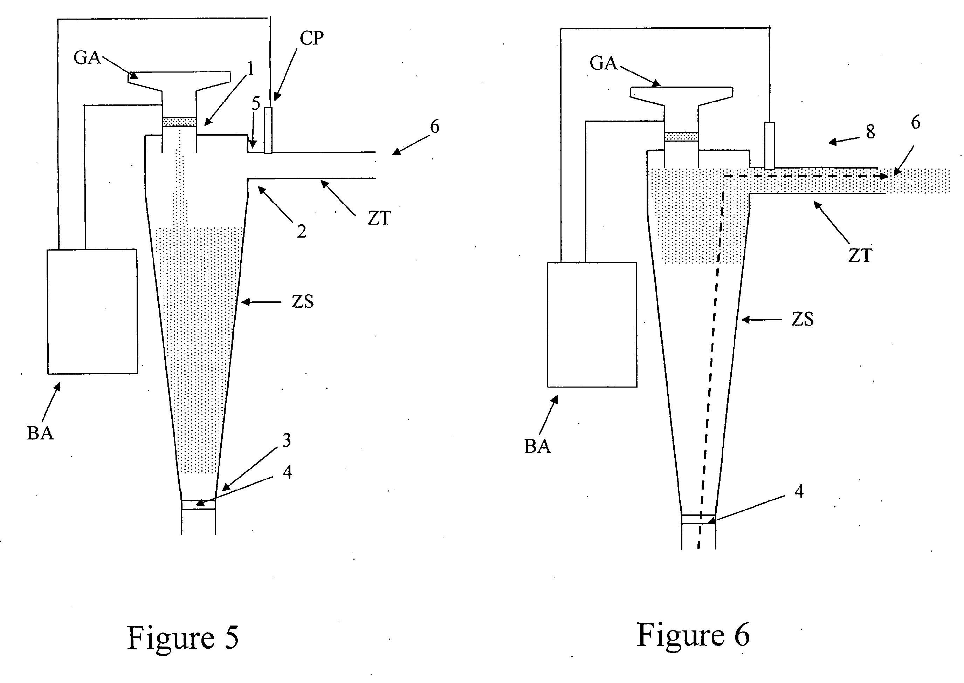

Une première configuration de l'appareil avec le dispositif de l'invention est illustrée aux figures 5 et 6, et concerne l'arrêt de la production de l'aérosol durant la totalité de la phase inspiratoire du patient dans le système à valve unique. Dans cette configuration, la zone de stockage (ZS) possède trois ouvertures. Une première ouverture (1) placée sur le haut de la zone de stockage (ZS)est destinée à recevoir un générateur d'aérosol (GA) (par exemple un nébuliseur à membrane). Une deuxième ouverture (2) placée sur le coté latéral de la partie supérieure de la zone de stockage (ZS) est destinée à recevoir la zone de transport (ZT) vers le patient. Une troisième ouverture (3) placée sur l'extrémité basse est destinée à recevoir une valve (4). La zone de transport (ZT) est une pièce cylindrique possédant deux ouvertures. L'ouverture (5) de la zone de transport (ZT) est connectée à l'ouverture (2) de la zone de stockage (ZS) et à un capteur de débit ou de pression (CP). L'ouverture (6) de la zone de transport (ZT) est connectée au patient. Le capteur de débit ou de pression (CP) est relié au boîtier d'alimentation (BA), lui-même relié au générateur d'aérosol (GA) permettant son fonctionnement. Dans cette configuration, durant la phase expiratoire et les pauses respiratoires (Figure 5), la valve (4) est fermée. Durant la phase expiratoire et les pauses respiratoires, le capteur de débit ou de pression (CP) ne détecte pas de mouvement d'air, l'aérosol est alors produit et est stocké dans la zone de stockage (ZS) pour la prochaine inspiration.

Durant la phase inspiratoire (Figure 6), la valve (4) est ouverte. L'air pénétrant dans la zone de stockage (ZS) par l'intermédiaire de la valve (4) traverse la zone de stockage (ZS) de bas en haut pour transporter l'aérosol vers la zone de transport (ZT). L'aérosol est ensuite transporté depuis la zone de transport (ZT) vers la bouche du patient. Durant la phase inspiratoire, le capteur de débit ou de pression (CP) détecte le mouvement d'air, l'aérosol n'est pas produit.

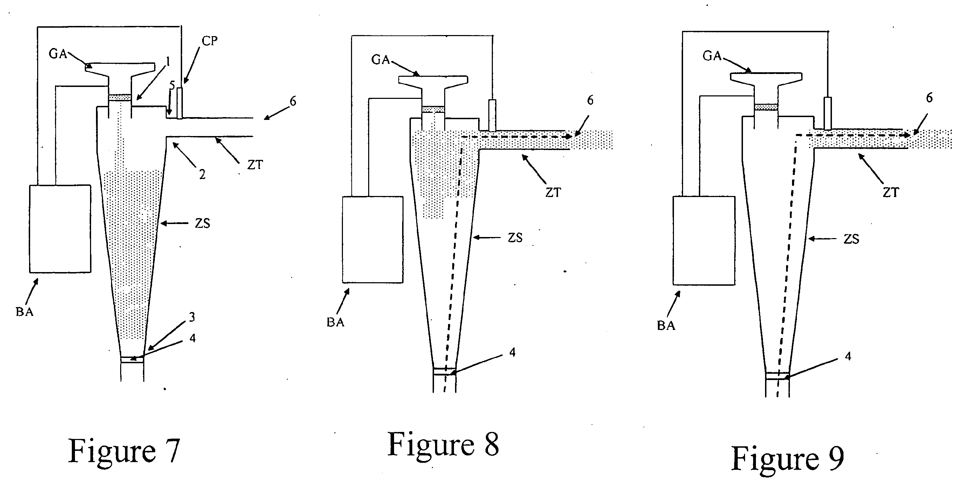

Une deuxième configuration de l'appareil avec le dispositif de l'invention est illustrée aux figures 7, 8 et 9, et concerne l'arrêt de la production de l'aérosol lors de la deuxième partie de la phase inspiratoire du patient dans le système à valve unique. Dans cette configuration, la zone de stockage (ZS) possède trois ouvertures. Une première ouverture (1) placée sur le haut de la zone de stockage (ZS)est destinée à recevoir un générateur d'aérosol (GA) (par exemple un nébuliseur à membrane). Une deuxième ouverture (2) placée sur le coté latéral de la partie supérieure de la zone de stockage (ZS) est destinée à recevoir la zone de transport (ZT) vers le patient. Une troisième ouverture (3) placée sur l'extrémité basse est destinée à recevoir une valve (4). La zone de transport (ZT) est une pièce cylindrique possédant deux ouvertures. L'ouverture (5) de la zone de transport (ZT) est connectée à l'ouverture (2) de la zone de stockage (ZS) et à un capteur de débit ou de pression (CP). L'ouverture (6) de la zone de transport (ZT) est connectée au patient. Le capteur de débit ou de pression (CP) est relié au boîtier d'alimentation (BA), lui-même relié au générateur d'aérosol (GA) permettant son fonctionnement. Dans cette configuration, durant la phase expiratoire et les pauses respiratoires (Figure 7), la valve (4) est fermée. Durant la phase expiratoire et les pauses respiratoires, le capteur de débit ou de pression (CP) ne détecte pas de mouvement d'air, l'aérosol est alors produit et est stocké dans la zone de stockage (ZS) pour la prochaine inspiration.

Durant la première partie du temps inspiratoire (Figure 8), la valve (4) est ouverte. L'air pénétrant dans la zone de stockage (ZS) par l'intermédiaire de la valve (5) traverse la zone de stockage (ZS) de bas en haut pour transporter l'aérosol vers la zone de transport (ZT). L'aérosol est ensuite transporté depuis la zone de transport (ZT) vers la bouche du patient. Durant cette première partie de la phase inspiratoire, le capteur de débit ou de pression (CP) détecte le mouvement d'air, l'aérosol est produit.

Durant la deuxième partie du temps inspiratoire (Figure 9), la valve (4) est toujours ouverte. L'air pénétrant dans la zone de stockage (ZS) par l'intermédiaire de la valve (4) traverse la zone de stockage (ZS) préalablement vidée d'aérosol vers la zone de transport (ZT). L'air est ensuite dirigé depuis la zone de transport (ZT) vers la bouche du patient. Durant cette deuxième partie du temps inspiratoire, le capteur de débit ou de pression (CP) détecte le mouvement d'air, l'aérosol n'est pas produit

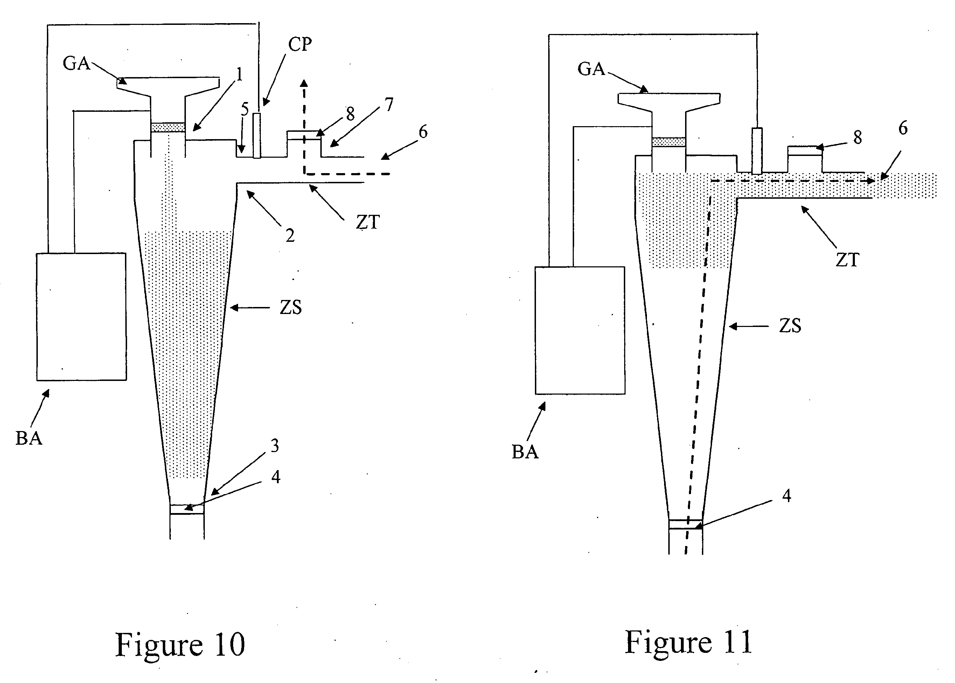

Une troisième configuration de l'appareil avec le dispositif de l'invention est illustrée aux figures 10 et 11, et concerne l'arrêt de la production de l'aérosol durant la totalité de la phase inspiratoire du patient dans un système à double valve. Dans cette configuration, la zone de stockage (ZS) possède trois ouvertures. Une première ouverture (1) placée sur le haut de la zone de stockage (ZS)est destinée à recevoir un générateur d'aérosol (GA) (par exemple un nébuliseur à membrane). Une deuxième ouverture (2) placée sur le coté latéral de la partie supérieure de la zone de stockage (ZS) est destinée à recevoir la zone de transport (ZT) vers le patient. Une troisième ouverture (3) placée sur l'extrémité basse est destinée à recevoir une valve (4). La zone de transport (ZT) est une pièce en forme de T à section circulaire possédant trois ouvertures. L'ouverture (5) de la zone de transport (ZT) est connectée à l'ouverture (2) de la zone de stockage (ZS) et à un capteur de débit ou de pression (CP). L'ouverture (6) de la zone de transport (ZT) est connectée au patient La troisième ouverture (7) située sur la zone de transport (ZT) est destinée à recevoir une valve (8). Le capteur de débit ou de pression (CP) est relié au boîtier d'alimentation (BA), lui-même relié au générateur d'aérosol (GA) permettant son fonctionnement. Dans cette configuration, durant la phase expiratoire et les pauses respiratoires (Figure 9), la valve (4) est fermée et la valve (8) est ouverte. L'air expiré par le patient ne passe pas par la zone de stockage (ZS) mais est expulsé hors de la zone de transport (ZT) par la valve (8). Durant la phase expiratoire et les pauses respiratoires, le capteur de débit ou de pression (CP) ne détecte pas de mouvement d'air, l'aérosol est alors produit et est stocké dans la zone de stockage (ZS) pour la prochaine inspiration.

Durant la totalité de la phase inspiratoire (Figure 11), la valve (4) est ouverte et la valve (8) est fermée. L'air pénétrant dans la zone de stockage (ZS) par l'intermédiaire de la valve (4) traverse la zone de stockage (ZS) de bas en haut pour transporter l'aérosol vers la zone de transport (ZT). L'aérosol est ensuite transporté depuis la zone de transport (ZT) vers la bouche du patient. Durant la phase inspiratoire, le capteur de débit ou de pression (CP) détecte le mouvement d'air, l'aérosol n'est pas produit.

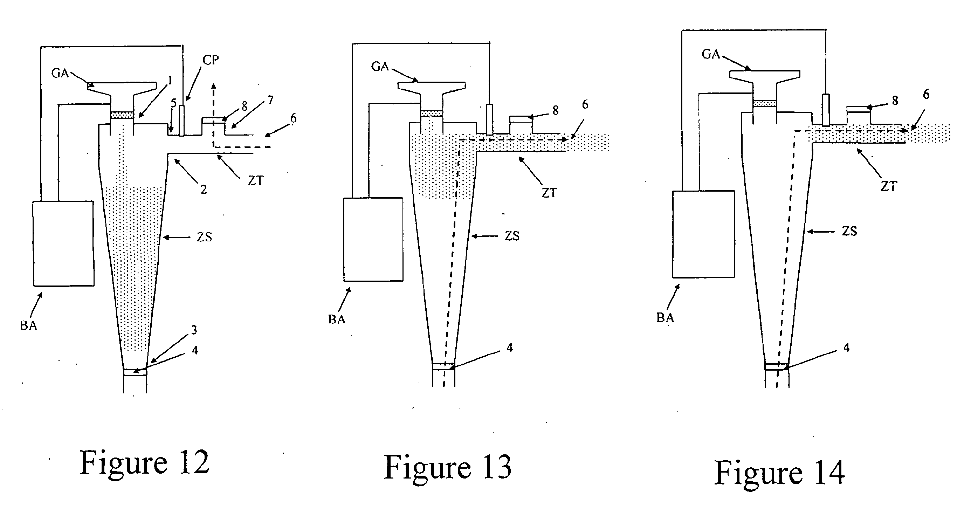

Une quatrième configuration de l'appareil avec un dispositif non couvert par l'invention est illustrée aux figures 12, 13 et 14 et concerne l'arrêt de la production de l'aérosol lors de la deuxième partie du temps inspiratoire du patient dans le système à double valve. Dans cette configuration, la zone de stockage (ZS) possède trois ouvertures. Une première ouverture (1) placée sur le haut de la zone de stockage (ZS)est destinée à recevoir un générateur d'aérosol (GA) (par exemple un nébuliseur à membrane). Une deuxième ouverture (2) placée sur le coté latéral de la partie supérieure de la zone de stockage (ZS) est destinée à recevoir la zone de transport (ZT) vers le patient. Une troisième ouverture (3) placée sur l'extrémité basse est destinée à recevoir une valve (4). La zone de transport (ZT) est une pièce en forme de T à section circulaire possédant trois ouvertures. L'ouverture (5) de la zone de transport (ZT) est connectée à l'ouverture (2) de la zone de stockage (ZS) et à un capteur de débit ou de pression (CP). L'ouverture (6) de la zone de transport (ZT) est connectée au patient. La troisième ouverture (7) située sur la zone de transport (ZT) est destinée à recevoir une valve (8). Le capteur de débit ou de pression (CP) est relié au boîtier d'alimentation (BA), lui-même relié au générateur d'aérosol (GA) permettant son fonctionnement. Dans cette configuration, durant la phase expiratoire et les pauses respiratoires (Figure 12), la valve (4) est fermée et la valve (8) est ouverte. L'air expiré par le patient ne passe pas par la zone de stockage (ZS) mais est expulsé hors de la zone de transport (ZT) par la valve (8). Durant la phase expiratoire et les pauses respiratoires, le capteur de débit ou de pression (CP) ne détecte pas de mouvement d'air, l'aérosol est alors produit et est stocké dans la zone de stockage (ZS) pour la prochaine inspiration.

Durant la première partie du temps inspiratoire de la phase inspiratoire (Figure 13), la valve (4) est ouverte et la valve (8) est fermée. L'air pénétrant dans la zone de stockage (ZS) par l'intermédiaire de la valve (4) traverse la zone de stockage (ZS) de bas en haut pour transporter l'aérosol vers la zone de transport (ZT). L'aérosol est ensuite transporté depuis la zone de transport (ZT) vers la bouche du patient. Durant cette première partie de la phase inspiratoire, le capteur de débit ou de pression (CP) détecte le mouvement d'air, l'aérosol est produit

Durant la deuxième partie du temps inspiratoire (Figure 14), la valve (4) est toujours ouverte. L'air pénétrant dans la zone de stockage (ZS) par l'intermédiaire de la valve (4) traverse la zone de stockage (ZS) préalablement vidée d'aérosol vers la zone de transport (ZT). L'air est ensuite dirigé depuis la zone de transport (ZT) vers la bouche du patient. Durant cette deuxième partie du temps inspiratoire, le capteur de débit ou de pression (CP) détecte le mouvement d'air, l'aérosol n'est pas produit.

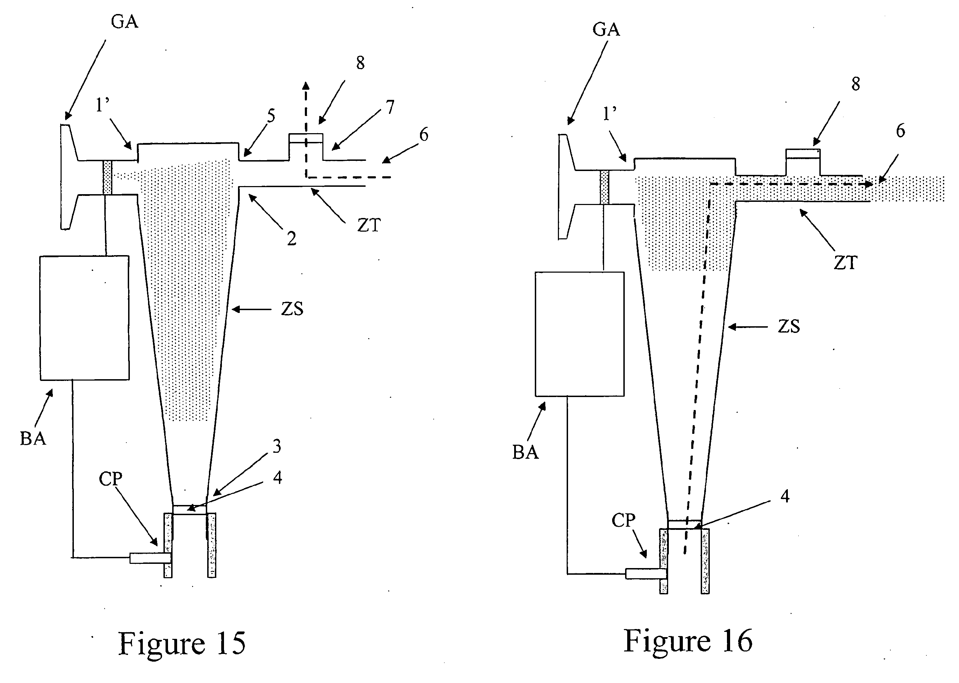

Une cinquième configuration de l'appareil avec le dispositif de l'invention est illustrée aux figures 15 et 16, et concerne l'arrêt de la production de l'aérosol lors de la totalité de la phase inspiratoire du patient dans l'appareil avec un système à double valve. Dans cette configuration, la zone de stockage (ZS) possède trois ouvertures. Une première ouverture (1') placée sur le coté latéral de la partie supérieure de la zone de stockage (ZS) est destinée à recevoir un générateur d'aérosol (GA) (par exemple un nébuliseur à membrane). Une deuxième ouverture (2) placée sur le coté latéral de la partie supérieure de la zone de stockage (ZS) et opposée à l'ouverture (1') est destinée à recevoir la zone de transport (ZT) vers le patient. Une troisième ouverture (3) placée sur l'extrémité basse est destinée à recevoir une valve (4) et est connectée à un capteur de débit ou de pression (CP). La zone de transport (ZT) est une pièce en forme de T à section circulaire possédant trois ouvertures. L'ouverture (5) de la zone de transport (ZT) est connectée à l'ouverture (2) de la zone de stockage (ZS). L'ouverture (6) de la zone de transport (ZT) est connectée au patient. La troisième ouverture (7) située sur la zone de transport (ZT) est destinée à recevoir une valve (8). Le capteur de débit ou de pression (CP) est relié au boîtier d'alimentation (BA), lui-même relié au générateur d'aérosol (GA) permettant son fonctionnement. Dans cette configuration, durant la phase expiratoire et les pauses respiratoires (Figure 15), la valve (4) est fermée et la valve (8) est ouverte. L'air expiré par le patient ne passe pas par la zone de stockage (ZS) mais est expulsé hors de la zone de transport (ZT) par la valve (8). Durant la phase expiratoire et les pauses respiratoires, le capteur de débit ou de pression (CP) ne détecte pas de mouvement d'air, l'aérosol est alors produit et est stocké dans 1a zone de stockage (ZS) pour la prochaine inspiration.

Durant la phase inspiratoire (Figure 16), la valve (4) est ouverte et la valve (8) est fermée. L'air pénétrant dans la zone de stockage (ZS) par l'intermédiaire de la valve (4) traverse la zone de stockage (ZS) de bas en haut pour transporter l'aérosol vers la zone de transport (ZT). L'aérosol est ensuite transporté depuis la zone de transport (ZT) vers la bouche du patient. Durant la phase inspiratoire, le capteur de débit ou de pression (CP) détecte le mouvement d'air, l'aérosol n'est pas produit.

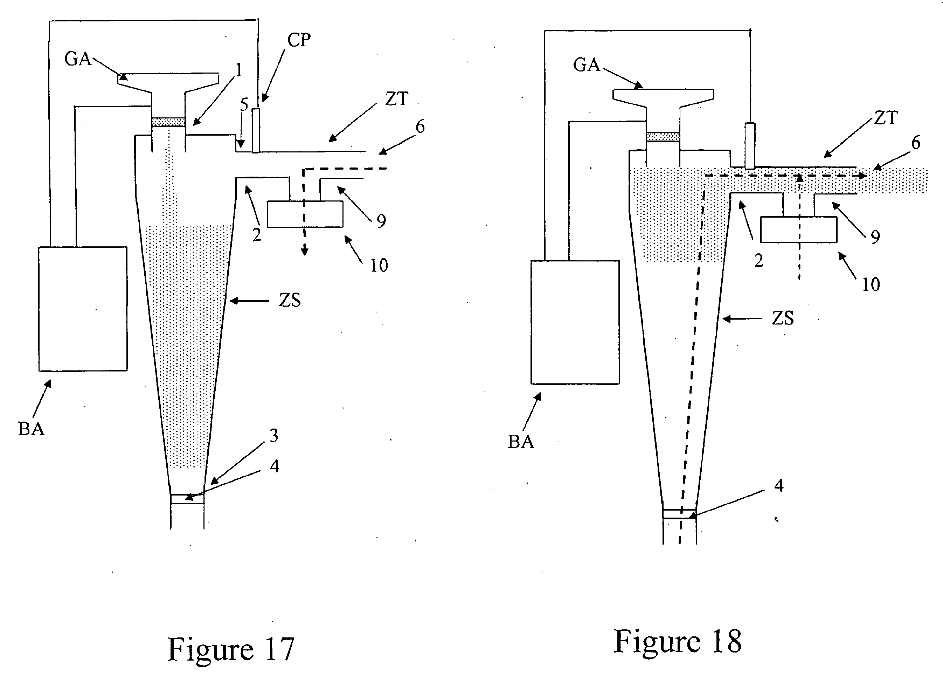

Une sixième configuration de l'appareil avec le dispositif de l'invention est illustrée aux figures 17 et 18, et concerne l'arrêt de la production de l'aérosol durant la totalité de la phase inspiratoire du patient dans un système à valve unique avec filtre expiratoire. Dans cette configuration, la zone de stockage (ZS) possède trois ouvertures. Une première ouverture (1) placée sur le haut de la zone de stockage (ZS)est destinée à recevoir un générateur d'aérosol (GA) (par exemple un nébuliseur à membrane). Une deuxième ouverture (2) placée sur le coté latéral de la partie supérieure de la zone de stockage (ZS) est destinée à recevoir la zone de transport (ZT) vers le patient. Une troisième ouverture (3) placée sur l'extrémité basse est destinée à recevoir une valve (4), La zone de transport (ZT) est une pièce en forme de T à section circulaire possédant trois ouvertures. L'ouverture (5) de la zone de transport (ZT) est connectée à l'ouverture (2) de la zone de stockage (ZS) et à un capteur de débit ou de pression (CP). L'ouverture (6) de la zone de transport (ZT) est connectée au patient. La troisième ouverture (9) située sur la zone de transport (ZT) est destinée à recevoir un filtre (10). Le capteur de débit ou de pression (CP) est relié au boîtier d'alimentation (BA), lui-même relié au générateur d'aérosol (GA) permettant son fonctionnement. Dans cette configuration, durant la phase expiratoire et les pauses respiratoires (Figure 17), la valve (4) est fermée. L'air expiré par le patient ne passe pas par la zone de stockage (ZS) mais est expulsé hors de la zone de transport (ZT) par le filtre (10). Durant la phase expiratoire et les pauses respiratoires, le capteur de débit ou de pression (CP) ne détecte pas de mouvement d'air, l'aérosol est alors produit et est stocké dans la zone de stockage (ZS) pour la prochaine inspiration.

Durant la totalité de la phase inspiratoire (Figure 18), la valve (4) est ouverte. L'air pénétrant dans la zone de stockage (ZS) par l'intermédiaire de la valve (4) traverse la zone de stockage (ZS) de bas en haut pour transporter l'aérosol vers la zone de transport (ZT). L'air pénètre également par le filtre (10)avec un débit moins important que par l'ouverture (3) selon la résistance du filtre ou de l'ouverture (9) au passage de l'air. L'aérosol est ensuite transporté depuis la zone de transport (ZT) vers la bouche du patient. Durant la phase inspiratoire, le capteur de débit ou de pression (CP) détecte le mouvement d'air, l'aérosol n'est pas produit.

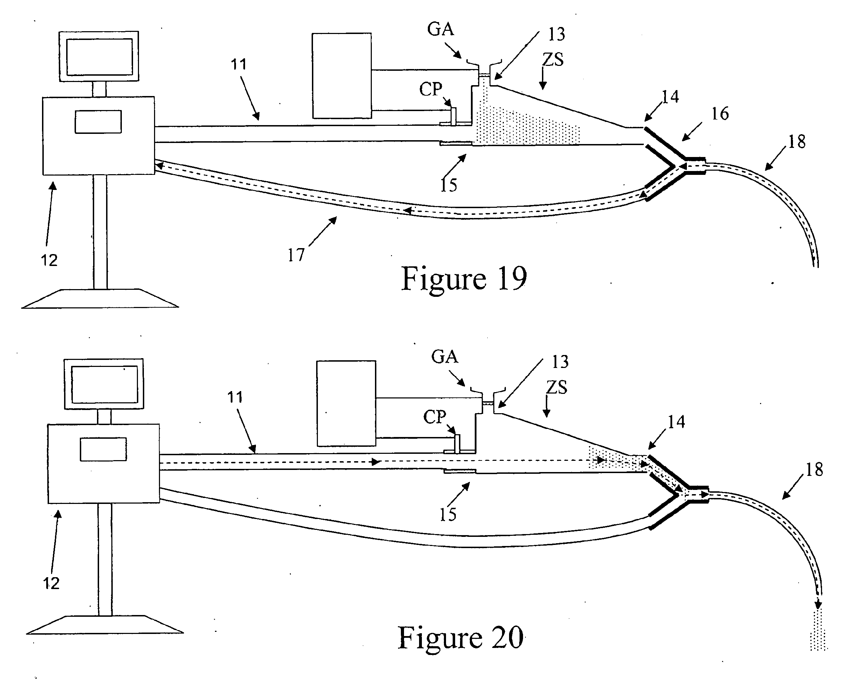

Une septième configuration de l'appareil incluant le dispositif de l'invention est illustré aux figures 19 et 20, et concerne l'arrêt de la production de l'aérosol, durant la totalité de la phase inspiratoire du patient, lorsque le patient est ventilé mécaniquement par un respirateur. Dans cette configuration, la zone de stockage (ZS) possède trois ouvertures et est interposée sur le circuit inspiratoire (11) du respirateur (12). Une première ouverture (13) placée sur le haut de la zone de stockage (ZS) est destinée à recevoir un générateur d'aérosol (GA) (par exemple un nébuliseur à membrane), Une deuxième ouverture (14) placée sur le coté latéral de la zone de stockage (ZS) est destinée à recevoir la fin du circuit inspiratoire du respirateur (16). Une troisième ouverture (15) placée à l'opposé de l'ouverture 14 est destinée à recevoir un capteur de débit ou de pression (CP) et le circuit inspiratoire (11) du ventilateur mécanique. Le capteur de débit ou de pression (CP) est relié au boîtier d'alimentation (BA), lui-même relié au générateur d'aérosol (GA) permettant son fonctionnement. La fin du circuit inspiratoire et le circuit expiratoire sont reliés par une pièce en forme de Y (16), elle même connectée à une sonde d'intubation endotrachéale (18) permettant la ventilation contrôlée du patient.

Dans cette configuration, durant la phase expiratoire et les pauses respiratoires (Figure 19), l'air inspiré par le respirateur (expiré pour le patient) passe par le circuit expiratoire et donc ne traverse pas la zone de stockage (ZS). Durant cette phase, le capteur de débit ou de pression (CP) ne détecte pas de mouvement d'air, l'aérosol est alors produit et est stocké dans la zone de stockage (ZS).

Durant la totalité de la phase inspiratoire (Figure 20), l'air expiré par le respirateur (inspiré pour le patient) passe par le circuit inspiratoire et donc traverse la zone de stockage (ZS). Durant cette phase, le capteur de débit ou de pression (CP) détecte de mouvement d'air, le générateur d'aérosol (GA) ne produit plus d'aérosol.

L'aérosol est alors transporté depuis la zone de stockage (ZS) vers le patient.

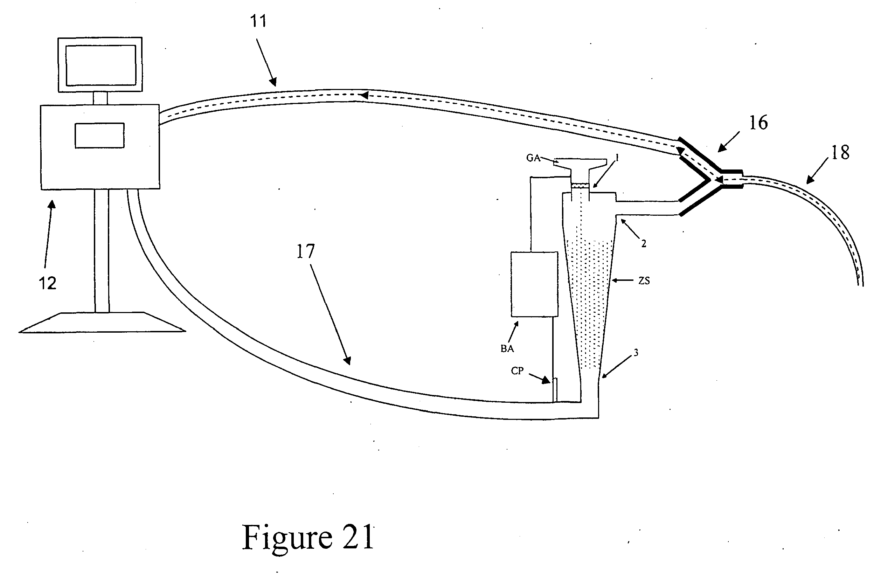

Une huitième configuration de l'appareil incluant un dispositif non couvert par l'invention est illustré aux figures 21, 22 et 23 et concerne l'arrêt de la production de l'aérosol uniquement durant la deuxième partie du temps inspiratoire, lorsque le patient est ventilé mécaniquement par un respirateur. Dans cette configuration, la zone de stockage (ZS) possède trois ouvertures et est interposée sur le circuit inspiratoire (11) du respirateur (12). Une première ouverture (1) placée sur le haut de la zone de stockage (ZS) est destinée à recevoir un générateur d'aérosol (GA) (par exemple un nébuliseur à membrane), Une deuxième ouverture (2) placée sur le coté latéral de la zone de stockage (ZS) est destinée à recevoir la fin du circuit inspiratoire du respirateur (16). Une troisième ouverture (3) placée en bas de la ZS est destinée à recevoir un capteur de débit ou de pression (CP) et le circuit inspiratoire (11) du ventilateur mécanique. Le capteur de débit ou de pression (CP) est relié au boîtier d'alimentation (BA), lui-même relié au générateur d'aérosol (GA) permettant son fonctionnement La fin du circuit inspiratoire et le circuit expiratoire sont reliés par une pièce en forme de Y (16), elle même connectée à une sonde d'intubation endotrachéale (18) permettant la ventilation contrôlée du patient.

Dans cette configuration, durant la phase expiratoire et les pauses respiratoires (Figure 21), l'air inspiré par le respirateur (expiré pour le patient) passe par le circuit expiratoire et donc ne traverse pas la zone de stockage (ZS). Durant cette phase, le capteur de débit ou de pression (CP) ne détecte pas de mouvement d'air, l'aérosol est alors produit et est stocké dans la zone de stockage (ZS).

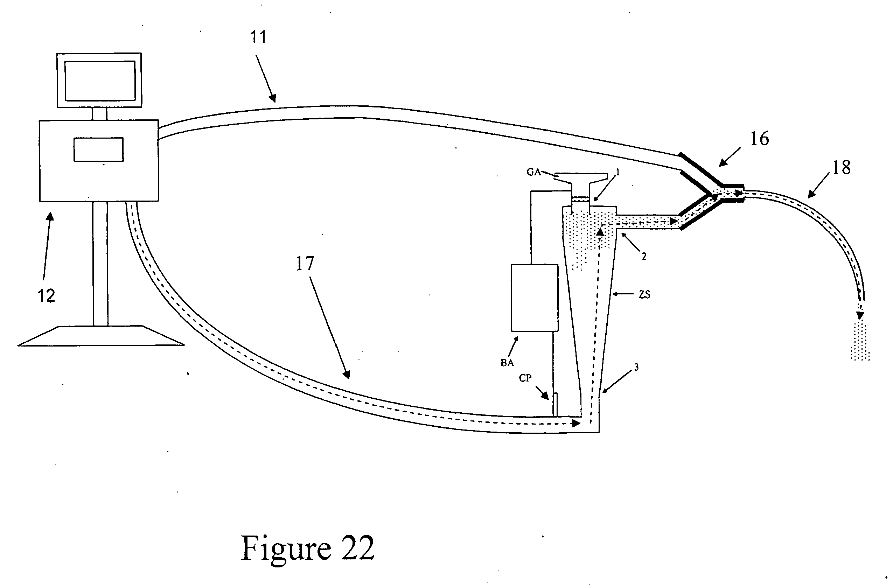

Durant la première partie du temps inspiratoire (Figure 22), l'air expiré par le respirateur (inspiré pour le patient) passe par le circuit inspiratoire et donc traverse la zone de stockage (ZS). L'aérosol est ensuite transporté depuis la zone de stockage (ZS) vers le patient. Durant cette première partie de la phase inspiratoire, le capteur de débit ou de pression (CP) détecte le mouvement d'air l'aérosol est produit.

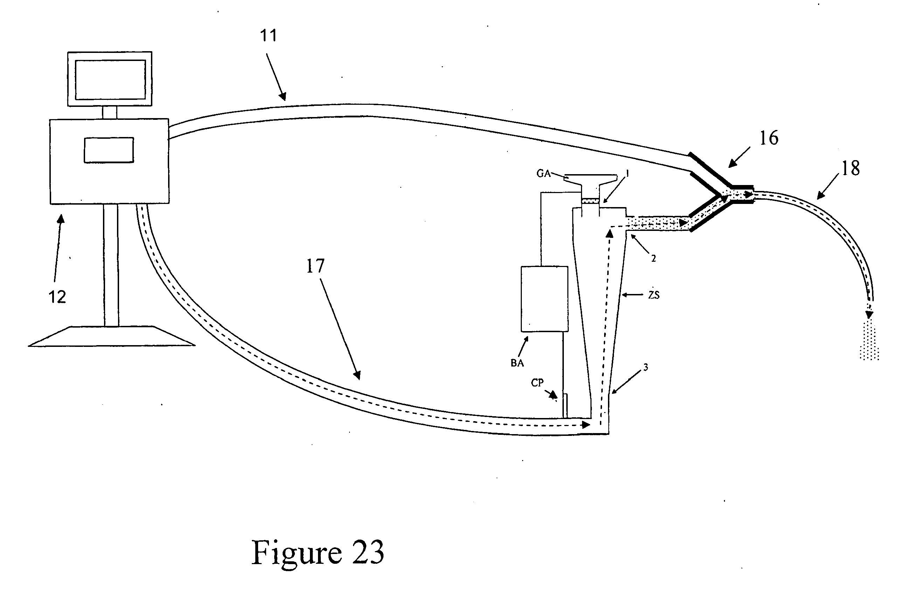

Durant la deuxième partie du temps inspiratoire (Figure 23), l'air expiré par le respirateur (inspiré pour le patient) passe par le circuit inspiratoire et donc traverse la zone de stockage (ZS). L'air est ensuite transporté depuis la zone de stockage (ZS) vers le patient Durant cette deuxième partie de la phase inspiratoire, le capteur de débit ou de pression (CP) détecte le mouvement d'air, l'aérosol n'est pas produit.

Dans les configurations précitées, l'orientation et la forme de la zone de stockage (ZS) peuvent varier, les figures ayant été décrites et citées à titre d'exemple. De même, l'emplacement des ouvertures sur la zone de stockage (ZS) et l'emplacement de la zone de transport (ZT) et du générateur d'aérosol (GA) peuvent varier en position.

En conclusion, la solution apparaît extrêmement avantageuse, car selon les tests effectués, il a été mesuré que la dose d'aérosol déposé dans les poumons du patient pouvait être augmentée jusqu'a 50% en comparaison des systèmes actuels.

L'appareil incluant le dispositif selon l'invention peut être utilisé non limitativement comme suit :
- en association avec un aérosol produit par une membrane ou tamis (mesh en anglosaxon)
- en association avec un aérosol produit par un nébuliseur pneumatique,
- en association avec un aérosol produit par un nébuliseur ultrasonique,
- en association avec un aérosol produit par tout autre nébuliseur (disque tournant, injection de liquide sous pression...)

L'appareil avec le dispositif d'amélioration de l'efficacité de dépôt d'aérosol selon l'invention est simple à réaliser et permet d'améliorer le dépôt d'aérosol dans les voies respiratoires par diminution de la fraction exhalée. Il peut être raccordé à un appareil d'assistance respiratoire ou utilisé en ambulatoire. Il améliore également le débit d'aérosol déposé dans les poumons en comparaison des systèmes produisant l'aérosol durant toute ou partie de la phase inspiratoire du patient.

Le dispositif possède également l'avantage de pouvoir contrôler la dose à administrer dans les poumons du patient. Par exemple dans des nouvelles thérapies comme la chimiothérapie par aérosol où la dose à administrer dans les poumons doit être contrôlée, l'appareil permet d'assurer un dépôt optimal du médicament dans les voies respiratoires. La quantité d'aérosol produit correspond à la quantité d'aérosol déposée dans les voies respiratoires.

Ainsi l'appareil avec le dispositif selon l'invention est particulièrement avantageux car il permet d'améliorer l'efficacité de dépôt de l'aérosol dans les voies respiratoires tout en réduisant la durée du traitement des systèmes comparables et actuellement commercialisés. L'invention est remarquable en ce que l'aérosol est produit dans une zone de stockage durant les phases expiratoires, les pauses inspiratoires et las pauses expiratoires du patient. Par exemple, lorsque le patient inspire dans le système, il n'y a plus de génération d'aérosol. Dans le cas où la zone de stockage (ZS) est un volume assez faible (V1) (inférieur au volume inspiré par le patient, 200 ml par exemple), seule une partie de l'air inhalé par le patient sera chargée d'aérosol. Par exemple, si un patient inhale 500ml d'air (Vt), les premiers 200ml d'air (V1) seront chargés d'aérosol et les 300 derniers ml (V2) d'air seront vides d'aérosol. Dans ces conditions, à la fin de l'inspiration, l'air contenu dans la région de conduction (RC) du patient sera dépourvu ou partiellement dépourvu d'aérosol. Or, la majorité de l'aérosol exhalé provient principalement de la région de conduction (RC) des voies aériennes du patient. Ainsi, l'air exhalé par le patient est appauvri en aérosol et l'efficacité de dépôt de l'aérosol est augmentée.

L'appareil avec le dispositif selon l'invention est également remarquable en ce qu'il améliore le débit d'aérosol déposé dans les poumons en comparaison des systèmes produisant l'aérosol durant une partie de la phase inspiratoire du patient. En effet, si l'on considère la respiration d'un adulte à 20 cycles respiratoires par minute et un rapport entre le temps inspiratoire et le temps expiratoire de ½, alors le temps inspiratoire est de 1 sec et le temps expiratoire de 2 sec. Avec les appareils actuels produisant l'aérosol durant la première moitié de l'inspiration, la durée de génération de l'aérosol sera par exemple de 0.5sec par inspiration. Avec la présente invention produisant l'aérosol durant la non inspiration, la durée de génération de l'aérosol sera de 2sec par inspiration soit un débit d'aérosol 4 fois plus important.

De plus, l'appareil avec le dispositif selon l'invention est particulièrement adapté au patient car il permet son fonctionnement dans toutes les situations de respiration. En effet, si l'on considère la séance d'inhalation avec un embout buccal, l'appareil décrit dans le brevet US4819629 ne permet pas de générer l'aérosol. En effet, si le patient expire par le nez, le capteur de pression placé sur la ligne expiratoire ne détecte pas le mouvement d'air et l'aérosol n'est pas généré durant la phase expiratoire du patient. Le système doit alors être utilisé avec un masque étanche recouvrant la bouche et les narines. Avec la présente invention, ce problème ne persiste plus. Le capteur de mouvement d'air étant placé sur le circuit inspiratoire du système, c'est l'inspiration du patient dans le système qui permet d'arrêter la production de l'aérosol. La présente invention permet donc un fonctionnement opérationnel avec un embout buccal. De la même façon, lorsque le patient réalise une pause respiratoire en fin d'expiration (Pe, Figure la) ou d'inspiration (Pi, Figure la), le brevet US4819629 ne permet pas la génération de l'aérosol (Figure 1d). Dans ces conditions la synchronisation n'est pas optimale et la durée de la séance s'en trouve augmentée. Avec la présente invention, lorsque le patient réalise une pause respiratoire en fin d'expiration ou d'inspiration, la présente invention permet la génération de l'aérosol et son stockage pour la prochaine inspiration. Dans ces conditions la synchronisation est optimale et la durée de la séance s'en trouve diminuée.

L'invention permet donc non seulement d'augmenter le rendement de l'appareil en termes de dépôt d'aérosol dans les poumons mais également le débit d'aérosol déposé dans les poumons. L'invention est également adaptée au patient car elle permet son fonctionnement optimal dans toutes les situations de respiration.

## Revendications

1. Appareil générateur d'aérosol pour liquide à fonctionnement automatique agencé pour améliorer l'efficacité de dépôt d'aérosol comprenant :
- un générateur d'aérosol (GA) de liquide sous forme de nébuliseur produisant un aérosol de façon continue
- une zone de stockage (ZS) de l'aérosol,
- une zone de transfert (ZT) de l'aérosol vers le patient,
- un dispositif à fonctionnement automatique permettant la génération de l'aérosol et son transfert dans la zone de stockage uniquement durant les phases de pauses inspiratoires, les phases expiratoires et les phases de pauses expiratoires ; et par l'arrêt de la génération de l'aérosol par le générateur d'aérosols dans la zone de stockage durant la totalité de l'inspiration du patient dans le système,
- une zone de stockage de l'aérosol produit contenant au minimum deux ouvertures dont l'une permet au minimum le passage de l'air et l'autre le passage de l'aérosol depuis la zone de stockage vers le patient n'autorisant pas le passage de l'air expiré par le patient à travers la zone de stockage,
- le dispositif étant un capteur placé sur le circuit inspiratoire de l'air induit par le patient et relié au générateur d'aérosol par un boîtier d'alimentation, ledit capteur ayant pour fonction d'identifier les phases d'inspiration pour produire l'aérosol dans la zone de stockage, durant la totalité de la phase de non inspiration du patient dans l'appareil et l'arrêt de la production d'aérosol lors de la totalité de l'inspiration du patient dans l'appareil.

2. Appareil générateur d'aérosol à fonctionnement automatique selon la revendication 1 **caractérisé en ce que** la zone de stockage (ZS) présente un volume proportionné au volume de l'inspiration du patient.

3. Appareil pour l'administration d'un aérosol, **caractérisé en ce qu'**il comprend un appareil générateur d'aérosol permettant l'amélioration de l'efficacité de dépôt de l'aérosol selon l'une quelconque des revendications 1 à 2 et un respirateur de ventilation mécanique.

4. Appareil pour l'administration d'un aérosol, **caractérisé en ce qu'**il comprend un appareil générateur d'aérosol permettant l'amélioration de l'efficacité de dépôt de l'aérosol, selon l'une quelconque des revendications 1 à 2 et un embout buccal.

5. Appareil pour l'administration d'un aérosol, **caractérisé en ce qu'**il comprend un appareil générateur d'aérosol permettant l'amélioration de l'efficacité de dépôt de l'aérosol, selon l'une quelconque des revendications 1 à 2 et un embout nasal.

## Claims

1. Automatic liquid aerosol generator arranged to improve aerosol deposit efficiency comprising:
- a liquid aerosol generator (GA) in the form of a nebulizer generating an aerosol continuously,
- a zone (ZS) for storing the aerosol,
- a zone (ZT) for conveying the aerosol to the patient,
- an automatic device for generating the aerosol, and conveying it to the storage zone solely during the inspiratory pause, expiratory and expiratory pause phases; with aerosol generation being stopped by the aerosol generator in the storage zone whenever the patient is inhaling from the system,
- a zone for storing the generated aerosol that contains at least two openings at least one of which lets through at least air, the other allowing aerosol to pass from the storage zone to the patient but not allowing the air exhaled by the patient to pass through the storage zone,
- the device being a sensor placed on the inspiratory circuit of the air induced by the patient and connected to the aerosol generator by a supply unit, the function of said sensor being to identify the inhalation phases in order to generate aerosol in the storage zone whenever the patient is not inhaling from the appliance, and cause aerosol generation to stop whenever the patient is inhaling from appliance.

2. Automatic aerosol generator as claimed in claim 1, **characterized in that** the storage zone (ZS) has a volume proportionate to the patient's inspiration volume.

3. Device for administering an aerosol, **characterized in that** it comprises an aerosol generator allowing improvement in aerosol deposit efficiency as claimed in any one of claims 1 to 2 and a mechanical ventilation respirator.

4. Device for administering an aerosol, **characterized in that** it comprises an aerosol generator allowing improvement in aerosol deposit efficiency, as claimed in any one of claims 1 to 2 and a mouthpiece.

5. Device for administering an aerosol, **characterized in that** it comprises an aerosol generator allowing improvement in aerosol deposit efficiency, as claimed in any one of claims 1 to 2 and a nasal applicator.

## Patentansprüche

1. Automatisch arbeitender Aerosolerzeuger für Flüssigkeiten, der so ausgebildet ist, um die Effizienz des Aerosolauftrags zu verbessern, bestehend aus:
- einem Flüssigkeitsaerosolerzeuger (GA) in Form eines Verneblers, der kontinuierlich ein Aerosol erzeugt,
- einem Speicherbereich (ZS) des Aerosols,
- einem Transferbereich (ZT) des Aerosols zum Patienten,
- einer automatisch arbeitenden Vorrichtung für die Generierung des Aerosols und dessen Transfer in den Speicherbereich nur während der Einatmungspausenphasen, Ausatmungsphasen und Ausatmungspausenphasen; und für das Stoppen der Aerosolgenerierung durch den Aerosolerzeuger im Speicherbereich während der gesamten Einatmungsphase des Patienten im System,
- einem Speicherbereich des erzeugten Aerosols mit mindestens zwei Öffnungen, wovon eine mindestens den Durchstrom der Luft und die andere den Durchstrom des Aerosols vom Speicherbereich zum Patienten ermöglicht, aber nicht den Durchstrom der vom Patienten ausgeatmeten Luft durch den Speicherbereich,
- wobei die Vorrichtung aus einem Sensor besteht, der an dem vom Patienten hervorgerufenen Einatmungsweg der Luft sitzt und über ein Netzgerät mit dem Aerosolerzeuger verbunden ist, wobei der Sensor die Aufgabe hat, die Einatmungsphasen zu erkennen, um während der gesamten Nichteinatmungsphase des Patienten im Gerät das Aerosol im Speicherbereich zu produzieren und während der gesamten Einatmungsphase des Patienten im Gerät die Aerosolproduktion zu stoppen.

2. Automatisch arbeitender Aerosolerzeuger nach Anspruch 1, **dadurch gekennzeichnet, dass** der Speicherbereich (ZS) ein dem Einatmungsvolumen des Patienten entsprechendes Volumen aufweist.

3. Gerät für die Verabreichung eines Aerosols, **dadurch gekennzeichnet, dass** es einen Aerosolerzeuger, der nach einem der Ansprüche 1 - 2 die Effizienz des Aerosolauftrags verbessert, und ein mechanisches Beatmungsgerät für die Belüftung umfasst.

4. Gerät für die Verabreichung eines Aerosols, **dadurch gekennzeichnet, dass** es einen Aerosolerzeuger, der nach einem der Ansprüche 1 - 2 die Effizienz des Aerosolauftrags verbessert, und ein Mundstück umfasst.

5. Gerät für die Verabreichung eines Aerosols, **dadurch gekennzeichnet, dass** es einen Aerosolerzeuger, der nach einem der Ansprüche 1 - 2 die Effizienz des Aerosolauftrags verbessert, und ein Nasenstück umfasst.
